# EUROPEAN PATENT SPECIFICATION

(11) **EP 1 496 118 B1**
(45) Date of publication and mention of the grant of the patent: **21.10.2009**
(21) Application number: 03717587.4
(22) Date of filing: 15.04.2003
(51) Int. Cl.: G01N 33/50, C12N 15/09, C12Q 1/02

(54) **METHOD OF MEASURING HUMAN CYP3A INDUCIBILITY**
VERFAHREN ZUR MESSUNG DER INDUZIERBARKEIT VON MENSCHLICHEM CYP3A
PROCEDE DE MESURE DE L'INDUCTIBILITE DU CYP3A HUMAIN

(30) Priority: 15.04.2002 JP 2002112364
(43) Date of publication of application: 12.01.2005
(73) Proprietor: Yamazoe, Yasushi, Sendai-shi, Miyagi 981-0952 (JP); Sekisui Medical Co., Ltd., Tokyo 103-0027 (JP)
(72) Inventor: YAMAZOE, Yasushi, SENDAI-SHI, Miyagi 981-0952 (JP); NAGATA, Kiyoshi, SENDAI-SHI, Miyagi 981-0952 (JP)
(74) Representative: Hartz, Nikolai
(86) International application number: PCT/JP2003/004761
(87) International publication number: WO 2003/087365

(56) References cited:
- WO-A-01/18013
- WO-A-01/20025
- WO-A-02/08451
- YASUSHI YAMAZOE: 'Yakubutsu taishano no kojinsa no gen'in to naru kakunai tensha chosetsukei no kaiseki to sono yosoku' UEHARA KINEN SEIMEI KAGAKU ZAIDAN KENKYU HOKOKUSHU vol. 15, 2001, pages 108 - 109, XP002969416
- NAGATA K. ET AL.: 'Molecular mechanism of human CYP3A4 induction' XENOBIO. METABOL. AND DISPOS. vol. 16, no. 5, 2001, pages 485 - 490, XP002969417
- FURUKAWA M. ET AL.: 'Adenovirus vector-mediated reporter system for in vivo analyses of human CYP3A4 gene activation' J. BIOCHEM. vol. 131, no. 1, January 2002, TOKYO, pages 71 - 78, XP002969418
- GOODWIN B. ET AL.: 'The orphan human pregnane X receptor mediates the transcriptional activation of CYP3A4 by rifampicin through a distal enhancer module' MOL. PHARMACOL. vol. 56, no. 6, 1999, pages 1329 - 1339, XP002227854
- PASCUSSI J.M. ET AL.: 'Evidence for the presence of a functional pregnane X receptor response element in the CYP3A7 promoter gene' BIOCHEM. BIOPHYS. RES. COMMUN. vol. 260, no. 2, 1999, pages 3277 - 381, XP000907111

## Description

### TECHNICAL FIELD

The present invention relates to a method for measuring a capacity for inducing a human drug-metabolizing enzyme, known as human CYP3A, easily and accurately. This invention also relates to a reagent useful for said measurement.

### BACKGROUND ART

WO 02/08451 discloses an assay for screening compounds capable of inducing drug metabolizing enzymes.

WO 01/20025 relates to the use of polymorhisms in the human CYP3A4 and CYP3A7 genes in diagnostic and therapeutic applications.

WO 01/18013 discloses prodrugs for liver specific drug delivery.

YASUSHI YAMAZOE, UEHARA KINEN SEIMEI KAGAKU ZAIDAN KENKYU HOKOKUSHU vol. 15, 2001, 108-109; NAGATA K. ET AL., XENOBIO. METABOL. AND DISPOS. vol. 16, no.5, 2001, 485-490, FURUKAWA M. et al., J. BIOCHEM. vol. 131, no. 1, January 2002, 71-78, and GOODWIN B. et al., MOL. PHARMACOL. vol. 56, no. 6, 1999, 1329-1339 relate to CYP3A4 and the pregnane X receptor.

PASCUSSI J.M. et al., BIOCHEM. BIOPHYS. RES. COMMUN. vol. 260, no. 2, 1999, 3277 - 381, relates to evidence for the presence of a functional pregnane X receptor response element in the CYP3A7 promoter gene.

Takeshita A. et al., European Journal of Endocrinology/European Federation of Endocrine Societies, vol. 145, no. 4, Oct 2001, 513-517) discloses a transcription assay for testing if bisphenol-A stimulates transcriptions of a specific gene.

Savas U. et al., Drug Metabolism and Disposition; the Biological Fate of Chemicals. vol. 28, no. 5, May 2000, 529-537 discloses activation of the rabbit pregnane X receptor by rifampicin.

Most of the drugs administered to human subjects undergo various metabolic pathways in the organs such as the liver. Among enzymes involved in drug metabolism, cytochrome P450, particularly CYP3A, is an enzyme that exerts the most influence on efficacy of a drug, occurrence of side effects, and disappearance of efficacy of the drug, and thus the measurement of CYP3A inducibility upon administration of a drug is an indispensable factor to be taken into account in the development of medical drugs. Some of these drugs have their own CYP3A inducibility, and therefore need to be measured and evaluated indivisually.

In conventional methods for measuring CYP3A inducibility, instead of measurement of CYP3A inducibility in humans, rats are used and a capacity for inducing CYP3A1 or CYP3A2, which corresponds to CYP3A in humans, is determined. However, since induction profile of CYP3A forms differs between humans and animals (such as rats) even with the same drug, it has been impossible to accurately evaluate the pharmacokinetic behavior of a drug in humans.

An object of the present invention is to provide an easy, accurate method for determining human CYP3A inducibility upon administration of a drug.

### DISCLOSURE OF THE INVENTION

The present inventors inserted a reporter gene and human nucleus receptor PXR (Pregnane X Receptor) cDNA into an adenovirus as a vector, and through use of the thus-prepared virus, measured human CYP3A inducibility in mice. Since the measurement system turned out to be a satisfactory assay system, they extended their research and found that dramatically accurate measurement-as compared with measurement attained by conventional methods or methods using a single plasmid-of human CYP3A inducibility can be realized by performing a reporter assay in a human cell culture system (*in vitro*) or a non-human animal (*in vivo*) incorporating the following two viruses; i.e., (A) an adenovirus which is used as a vector and engineered by incorporating thereto a reporter gene and at least 3 regions capable of binding to human PXR (hereinafter referred to simply as human PXR binding regions), and (B) an adenovirus which is used as a vector and engineered by incorporating thereto a human PXR cDNA. The present inventors also found that transformants capable of maintaining their traits after undergoing repeated subculture are present in a culture of human cells to which a specific DNA fragment has been incorporated-the DNA fragment constructed by inserting, into a plasmid vector (a), a detectable reporter gene and at least 3 human PXR binding regions falling within an untranslated region of a human CYP3A gene-and that use of such transformants further facilitates in vitro measurement of human CYP3A inducibility, thus leading to completion of the invention.

Accordingly, the present invention provides a method for measuring human CYP3A inducibility upon administration of a test drug, that a population of human cells cultured in a medium containing a test drug is infected with viruses (A) and (B), and subsequently expression level of the reporter gene described in relation to virus (A) is determined in the cultured human cells, wherein virus (A) is an adenovirus which is used as a vector and engineered by incorporating thereto a detectable reporter gene and at least 3 human PXR binding regions falling within an untranslated region of a human CYP3A gene, and virus (B) is an adenovirus which is used as a vector and engineered by incorporating thereto a human PXR cDNA, whereby the human cells are non-embryonic.

The present invention also provides a method for measuring human CYP3A inducibility upon administration of a test drug, which is defined by claim 1 and includes culturing transformed human cells in a medium containing a test drug, the transformed human cells being created by means of transfer of DNA the DNA constructed by inserting, into a plasmid vector (a), a detectable reporter gene and at least 3 human PXR binding regions falling within an untranslated region of a human CYP3A gene-and then measuring the expression level of the reporter gene, whereby the human cells are non-embryonic.

The present invention also provides a reagent for measuring human CYP3A inducibility, characterized by the features of claim 2 and comprising viruses (A) which is an adenovirus used as a vector and is engineered by incorporating thereto a detectable reporter gene and at least 3 specific human PXR binding regions falling within an untranslated region of a human CYP3A gene, and virus (B) which is an adenovirus which serves as a vector and engineered by incorporating thereto a human PXR cDNA.

A reagent for measuring human CYP3A inducibility, characterized by comprising transformed and cultured human cells which are created by means of transfer of DNA (a)-the DNA (a) constructed by inserting, into a plasmid vector, a detectable reporter gene and at least 3 human PXR binding regions falling within an untranslated region of a human CYP3A gene is also disclosed.

### BRIEF DESCRIPTION OF THE DRAWINGS

Fig. 1 illustrates a procedure for preparing a virus (A) (AdCYP3A4-362-7K);
Fig. 2 illustrates a procedure for preparing a virus (B) (AdhPXR);
Fig. 3 illustrates a procedure for measuring reporter activity by use of cultured cells;
Fig. 4 illustrates a procedure for measuring reporter activity in experimental animals;
Fig. 5 shows how reporter activity is affected by drugs when cultured cells are infected with an AdCYP3A4-362 virus (DMSO: dimethyl sulfoxide, DEX: dexamethasone, RIF: rifampicin, CLO: clotrimazole, concentration: 10 µM);
Fig. 6 shows how reporter activity is affected by drugs when cultured cells are simultaneously infected with virus (B) (AdhPXR) and virus (A) (AdCYP3A4-362-7K) (DMSO: dimethyl sulfoxide, DEX: dexamethasone, RIF: rifampicin, CLO: clotrimazole, concentration: 10 µM);
Fig. 7 shows how reporter activity and testosterone 6β-hydroxylation activity are affected by drugs in livers of mice to which AdCYP3A4-362 has been administered (DEX: dexamethasone, RIF: rifampicin, CLO: clotrimazole, dose: 100 mg/kg/day ×3);
Fig. 8 shows comparison between AdCYP3A4-362 and virus (A) (AdCYP3A4-362-7K) in terms of transcription induction in mice under co-administration with virus (B) (AdhPXR) (CLO: clotrimazole, Cont: control, dose: 100 mg/kg/day ×3);
Fig. 9 shows that administration of virus (B) (AdhPXR) alters mouse CYP3A induction so as to mimic human behavior (RIF: rifampicin, Cont: control, dose: 100 mg/kg/day ×3);
Fig. 10 shows how reporter activity expressed in mouse liver is affected by co-administration of virus (A) (AdCYP3A4-362-7K) and virus (B) (AdhPXR) (DEX: dexamethasone, RIF: rifampicin, CLO: clotrimazole, Cont: control, dose: 100 mg/kg/day ×3);
Fig. 11 illustrates a procedure for obtaining stable expression cell lines.
Fig. 12 shows how reporter activity is affected by treatment of several drugs in transformants (cells of consistent expression) to which DNA (a) has been transferred.

### BEST MODE FOR CARRYING OUT THE INVENTION

The method of the present invention for measuring human CYP3A uses the following two viruses:
(A) an adenovirus which is used as a vector and engineered by incorporating thereto a detectable reporter gene and at least 3 human PXR binding regions falling within an untranslated region of a human CY3A gene (hereinafter the virus may be referred to simply as virus (A)) as further defined by claim 1;
(B) an adenovirus which is used as a vector and engineered by incorporating thereto a human PXR cDNA (hereinafter the virus may be referred to simply as virus (B)).

The adenovirus vector which is used for creating virus (A) and virus (B) may be an adenovirus vector which is generally used as a vector in gene therapy. Preferably, those which contain no eukaryotic-cell-derived promoters or enhancers; for example, those from which early genes EIA·EIB are deleted, are preferred. Examples of commercial adenovirus vectors include Ad5dIX and AdEasy.

The detectable reporter gene used for obtaining virus (A) should be a gene coding for a detectable protein or a detectable enzyme. Examples of the detectable reporter gene include a GFP (green fluorescent protein) gene, a GUS (β-glucuronidase) gene, a LUS (lusiferase) gene, and a CAT (chloramphenicol acetyl transferase) gene. Examples of the commercial reporter gene include GFP, LUS, and CAT.

The above-mentioned "at least 3 human PXR binding regions falling within an untranslated region of a human CY3A gene" employed for obtaining virus (A) include as least a 7.7k (dNR-1) region, a 362 (ER-6) region, and a 7.6 to 7.4k (MIE) region, which fall within the untranslated region of a human CY3A gene. In the present invention, use of at least 3 human PXR binding regions is important. Use of only 1 or 2 such regions is not enough to induce expression of a human CY3A gene, preventing accurate measurement of human CYP3A inducibility.

As used herein, "human PXR" is referred to as a pregnane X receptor, which is a type of nucleus receptor. Human PXR is known to participate in expression of human CYP3A. However, it has remained unknown that the combination of at least 3 human PXR binding regions falling within an untranslated region of a human CYP3A gene is critical for expression of human CYP3A.

Human CYP3A includes human CYP3A4, CYP3A5, CYP3A7, and CYP3A43. CYP3A4 is the most important in terms of drug metabolism.

Virus (A) is prepared through the following processes. Firstly, a reporter virus is constructed by ligating at least 3 human PXR binding regions (for example, a 7.7k region, a 7.6 to 7.4k region, and a 362 region) upstream of an LUC gene. The resultant reporter plasmid is then inserted in the EIA·EIB region of an adenovirus vector.

Examples of the human PXR gene for constructing virus (B) include human PXR cDNA.

Virus (B) is prepared by, for example, preparing a human PXR cDNA according to the method described in Molecular Cloning (Maniatis, et al.) and inserting the resultant human PXR cDNA in the EIA·EIB region of an adenovirus vector.

Viruses (A) and (B) may be introduced into non-human animals to which a test drug has been administered or, according to the invention, to human cells cultured in a medium containing a test drug. The two viruses must be introduced in such a manner that their functions are exhibited simultaneously. The viruses may be introduced in the form of a solution mixture. Alternatively, one virus may be introduced first, followed by addition of the other virus, to thereby allow functions of the two viruses to develop simultaneously.

Examples of non-human animals include rats, mice, guinea pigs, rabbits, dogs, and monkeys. From the viewpoint of experimental handling, rats and mice are particularly preferred. The test drug is administered to these non-human animals through appropriate administration means generally used in clinical settings. Alternatively, peritoneal administration may be used. The dose of the test drug is determined in consideration of the clinical dose. Viruses (A) and (B) are preferably administered to non-human animals peritoneally or intravenously.

Examples of cultured human cells include HepG2, LS174T, Hela, and Caco-2. HepG2 and LS174T are particularly preferred. The cultured human cells are used as they are; i.e., without any treatment. Alternatively, the cells may be used after they are further cultured in a medium containing the test drug. Preferably, the cultured human cells are infected with viruses (A) and (B) through addition of the viruses to culture medium.

The transformed and cultured human cells used in the measurement of human CYP3A inducibility according to the present invention are obtained by transferring the following DNA to cultured human cells.
(a) DNA which is constructed by inserting, into a plasmid vector, a detectable reporter gene and at least 3 human PXR binding regions falling within an untranslated region of a human CYP3A gene (such DNA is referred to as DNA (a)).

Examples of the plasmid vector for preparing the DNA (a) include a vector having multi-cloning sites, such as pGL3 Basic Vector (Promega corporation) and a vector containing a GFP gene, such as pDSRed 2-1 (Clontech).

The detectable reporter gene and the at least 3 human PXR binding regions falling within an untranslated region of a human CYP3A gene for preparing DNA (a) may be any of those mentioned in relation to construction of virus (A).

DNA (a) is prepared through the following process. Firstly, a reporter vector is created by ligating at least 3 regions capable of binding to human PXR; for example a 7.7k region, a 7.6 to 7.4k region, and a 362 region. The resultant reporter vector is then inserted into the *Mlu*I-*Hind*III cleavage site within the multicloning region of plasmid vector having a reporter gene. In order to obtain a clone exhibiting stronger inducibility, it is preferable to use a DNA to which a plurality of plasmids, each having these regions and a reporter gene aligned in line, are ligated in tandem. More preferably, the number of plasmid is 5 or more, and even more preferably, 5 to 10.

In the present invention, the DNA (a) is transferred to cultured human cells. The transfer is preferably performed through, for example, the calcium phosphate method.

Examples of the cultured human cells useful in the present invention include HepG2, LS174T, Hela, and Caco-2. HepG2 and LS174T are particularly preferred. The transformants harboring DNA (a) can be selected by measuring the expression level of the reporter gene after subculturing.

In the case where transformed and cultured human cells that harbor and retain DNA (a) even after subculturing are used, human CYP3A inducibility of the test drug upon administration thereof can be determined by culturing such transformed and cultured human cells in a medium containing the test drug and then measuring the expression level of the reporter gene.

The expression level of the reporter gene in non-human animals is determined by, for example, measuring reporter activity in an organ such as the liver or the spleen 2 to 5 days after administration of viruses (A) and (B). The reporter activity in the liver is determined by, for example, homogenizing and centrifuging the liver removed from an animal and measuring the fluorescent intensity, reporter enzyme activity, or other reporter-related activities of the supernatant.

The expression level of the reporter gene in cultured human cells is determined by, for example, infecting the cells with viruses (A) and (B), and 2 to 4 days thereafter, homogenizing and centrifuging the infected cultured cells and measuring reporter activity of the supernatant.

Human CYP3A inducibility upon administration of a test drug can be estimated by comparing the obtained reporter activity with the reporter activity as measured in a test-drug non-administration group or in a test-drug-free medium culture group. Among the assay systems of the present invention using the viruses (A) and (B), *in vivo* systems employing non-human animals are particularly preferred.

### EXAMPLES

The present invention will next be described in more detail by way of examples, which should not be construed as limiting the invention thereto.

### Example 1

### A. Test Method

### (1) Construction of virus (A)

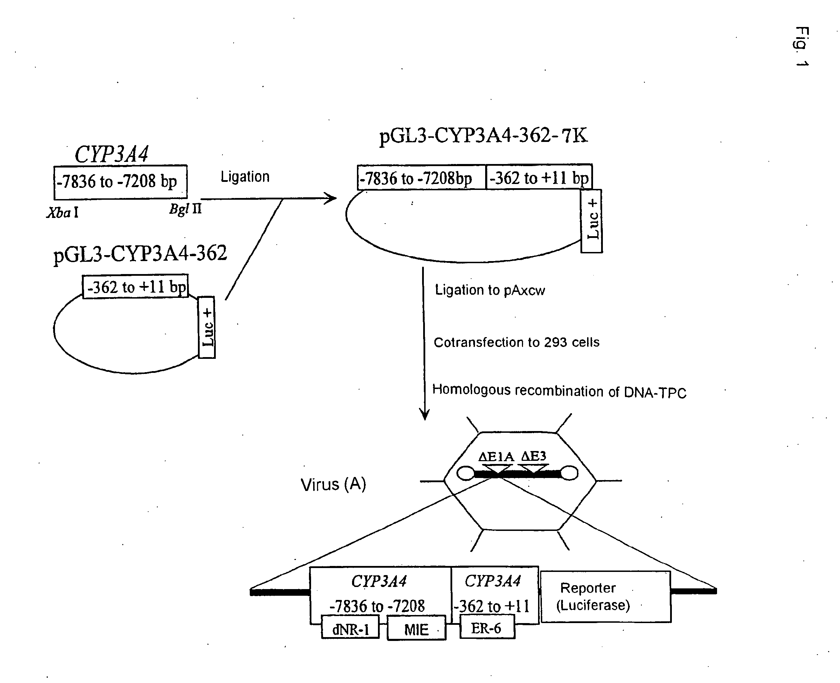

A recombinant adenovirus was constructed through the following process. Firstly, an upstream fragment (-362 to +11) of the CYP3A4 gene was isolated through PCR and then processed with restriction enzymes *Bgl* II and *Hind* III. Thereafter, the product was inserted into a promoterless firefly luciferase vector pGL3-Basic, to thereby prepare pGL3-CYP3A4-362. Separately, another upstream fragment (-7836 bp to -7208 bp) of the CYP3A4 gene, the fragment containing dNR-1 and the major inducible element (MIE), was isolated through PCR, subjected to subcloning, then inserted into the pGL3-CYP3A4-362 using the Bgl II and Xba I sites, to thereby prepare pGL3-CYP3A4-362-7K. The pGL3-CYP3A4-362 was excised and blunt-ended with a restriction enzyme *Xmn* I, and inserted in the blunt-ended Swa I site of a cosmid vector pAxcw. Subsequently, 293 cells were co-transfected with the cosmid vector having the above insert and a restriction-enzyme-treated adenovirus DNA-TPC, allowing homologous recombination to occur in the cells, to thereby construct virus (A) of interest (AdCYP3A4-362-7K) (Fig. 1). Separately, AdCYP3A4-362 virus, lacking the aforementioned region from - 7836 bp to -7208 bp, was also created.

### (2) Preparation of virus (B)

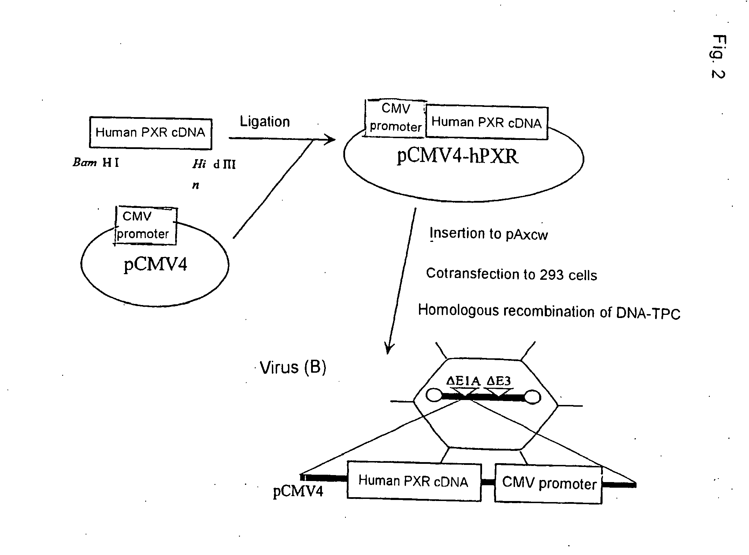

Through PCR, an hPXR cDNA was cloned from a human liver cDNA library, and inserted into the *Bam* HI and *Hind* III sites of a plasmid pCMV4 bearing a CMV promoter (the resultant plasmid will be referred to as pCMV4-hPXR). The plasmid pCMV4-hPXR was digested and blunt-ended with Sca I. Subsequently, a procedure similar to that described above in relation to construction of virus (A) was performed, to thereby obtain virus (B) (AdhPXR) (Fig. 2).

### (3) In vitro assay

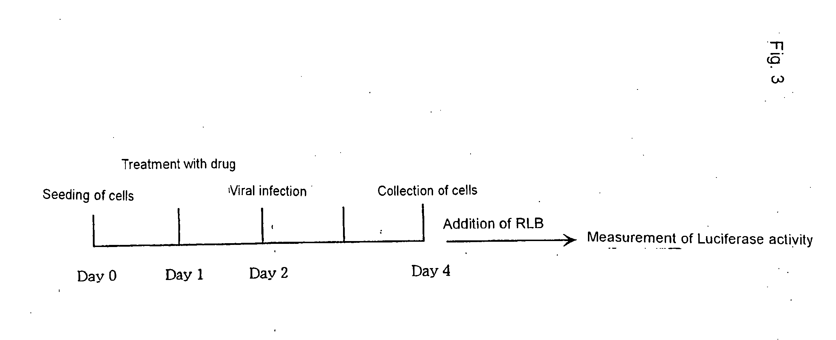

The assay was performed in a manner shown in Fig. 3. Briefly, cells were seeded in a 24-well plate in an amount of 2.0 × 10⁵ cells per well. On the following day; i.e., day 1, the cells were treated with a drug. On day 2, the drug-treated cells were infected with virus, and, 48 hours after viral infection, the cells were recovered. The recovered cells were lysed by use of a reporter lysis buffer (RLB), and the luciferase activity of the lysate was measured.

### (4) In vivo assay (Reference)

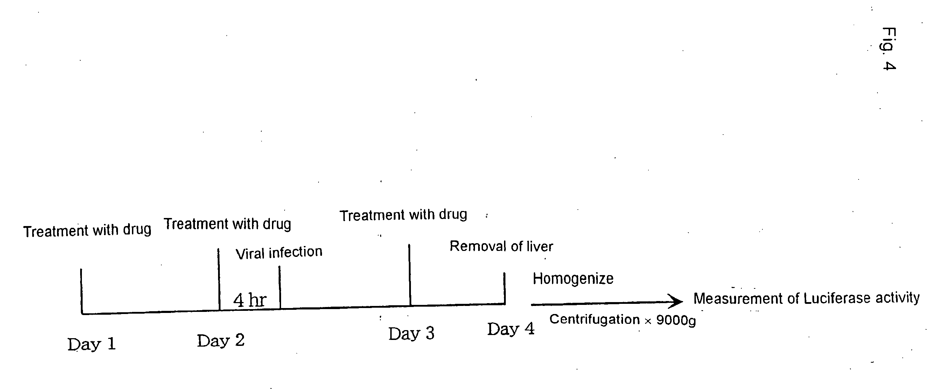

The initial plan for viral administration was an intravenous route through the tail vein. However, there were some cases where animals died within several hours following administration because of the virulence of the virus. Therefore, the route was changed to intraperitoneal administration so that the animal could not acutely respond to the virus. The drug was suspended in corn oil, and administered to each animal three times; i.e., one day before, four hours before, and one day after the virus administration, at a dose of 100 mg/kg each time. Two days after virus administration, each animal was dissected to remove the organ of interest (liver). The liver was homogenized and centrifuged at 9,000×g. Luciferase activity of the supernatant (S-9) was measured (Fig. 4).

### B. Test results

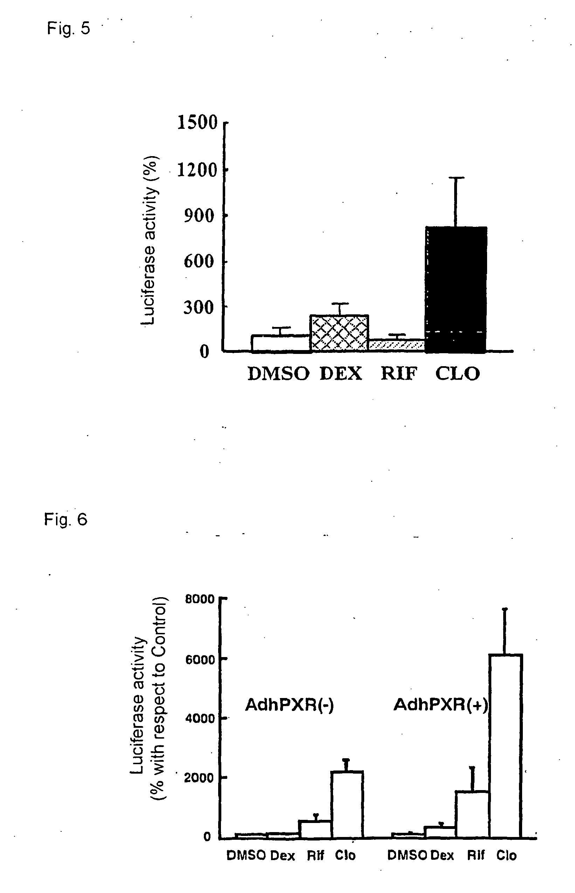

Fig. 5 shows luciferase activity, which is an index of CYP3A4 inducibility, elicited by different drugs in cultured cells infected with an AdCYP3A4-362 virus. Each of the test drugs (i.e., dexamethasone, rifampicin, and clotrimazole) was dissolved in DMSO, and the solution was added to the medium at a concentration of 10 µM. All these drugs are known to elicit CYP3A4 induction when administered to humans. In particular, rifampicin is known to be a typical human CYP3A4 inducer, because of its high specificity to humans. By contrast, dexamethasone is known to exhibit a strong induction of CYP3A forms in rats.

Assays employing human liver-cancer-derived cultured cells revealed an increase in luciferase activity in both cases of treatment with clotrimazole and treatment with dexamethasone. However, treatment with rifampicin did not show such an increase. The elevated luciferase activity levels observed were about eight times for clotrimazole treatment, and about two times for dexamethasone treatment.

Fig. 6 shows the results obtained from an *in vitro* reporter assay performed through use of virus (A) (AdCYP3A4-362-7K) under the same conditions as those of the above assay using the AdCYP3A4-362 virus. In response to dexamethasone, substantially no induction was obtained. In response to clotrimazole, a strong induction (21 times) was observed. Rifampicin was also found to exhibit an increased luciferase activity (five times), which was not observed in the above test employing the AdCYP3A4-362 virus. Next, virus (B) (AdhPXR) was employed to thereby cause strong expression of hPXR. As a result, an increase in luciferase activity by 10 to 30 times was obtained even in the cells which had not undergone drug treatment. Moreover, induction attributed to drug treatment was also enhanced. Specifically, when cells were treated with dexamethasone, luciferase activity was three times higher than that in the case of non-treatment, and with rifampicin and clotrimazole, luciferase activity was found to be 15 times and 60 times, respectively, higher than corresponding values obtained from non-treatment, revealing that the degree of transcription activation attained when cells are infected with virus (B) (AdhPXR) is higher than that attained when cells are not infected with virus (B).

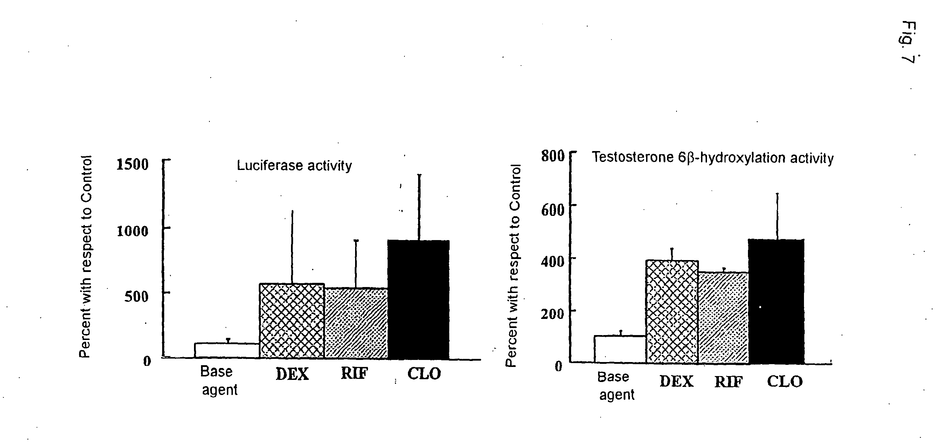

Next, an *in vivo* assay was carried out for evaluation of inducibility through use of ddY male mice. Each test drug was intraperitoneally administered once a day for three days, at a dose of 100 mg/kg each time. Four hours after the second drug administration, AdCYP3A4-362 virus was intraperitoneally administered to each mouse. Two days after the virus administration, the liver of the mouse was weighed, and the luciferase activity and the testosterone 6β-hydroxylation activity in the liver were measured. Each of the drugs (i.e., dexamethasone, rifampicin, and clotrimazole) was used as a suspension in corn oil. By administration of any of the inducing agents (dexamethasone, rifampicin, and clotrimazole), luciferase activity in the mouse liver S-9 was found to increase as compared with the control; i.e., by about five times in the cases of dexamethasone and rifampicin, and by about nine times in the case of clotrimazole. In addition, an increase in liver weight and an increase in testosterone 6β-hydroxylation activity were observed in all the inducer-treated groups. Increases in testosterone 6β-hydroxylation activity and increases in luciferase activity as determined in inducer-treated mouse livers were found to be well-correlated (Fig. 7).

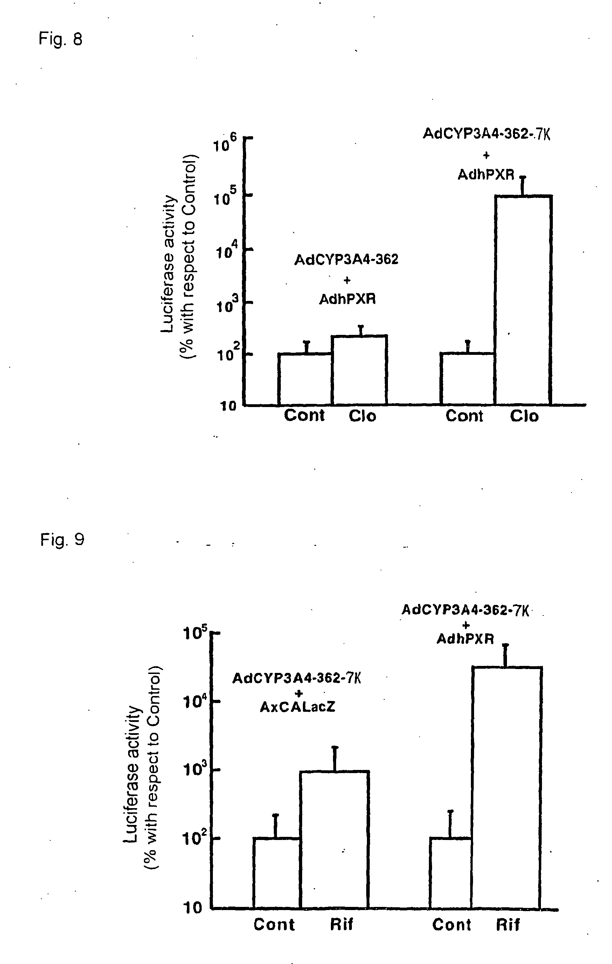

As described above, when cultured cells which had been coinfected with virus (A) (AdCYP3A4-362-7K) and virus (B) (AdhPXR) were subjected to drug treatment, CYP3A was found to be induced. Therefore, through viral infection of mice and subsequent administration of clotrimazole, level of CYP3A inducibility was compared between AdCYP3A4-362 virus and virus (A) (AdCYP3A4-362-7K). As a result, similar to the results obtained on cultured cells, mice which had been infected with virus (A) (AdCYP3A4-362-7K) were found to exhibit higher transcription activation than that exhibited by mice which had been infected with AdCYP3A4-362. The attained inducibility was found to be greatly higher than that obtained when cultured cells were used (1,000 times, see Fig. 8)

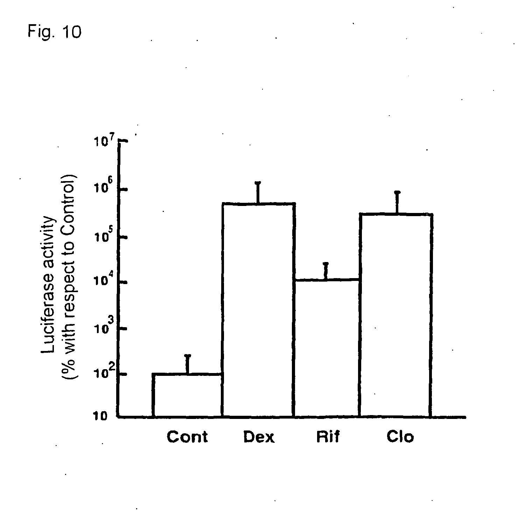

Next, CYP3A induction by rifampicin and effect of strong expression of hPXR were investigated after mice were co-infected with virus (A) (AdCYP3A4-362-7K) and virus (B) (AdhPXR). To a group to which virus (B) (AdhPXR) had not been administered, AxCALacZ used as a control virus was administered so as to equalize the viral dose. The mice which had been treated with rifampicin exhibited high transcription activation (about nine times) as compared with the control virus group, whereas the mice which had been co-infected with virus (B) (AdhPXR) and administered with rifampicin exhibited a considerably high transcription activation (about 320 times). Thus, by causing expression of human PXR, human CYP3A4 inducibility in response to rifampicin was obtained in mouse liver (Fig. 9). Moreover, in order to compare dexamethasone, rifampicin, and clotrimazole in terms of effect as an inducer, mice were co-infected with virus (AdCYP3A4-362-7K) and virus (B) (AdhPXR), and the three inducers were administered to the mice. Any of the inducers exhibited high inducibility, although rifampicin exhibited slightly lower inducibility than those of the others (Fig. 10).

### Example 2

### (1) Isolation of stable expression cell line

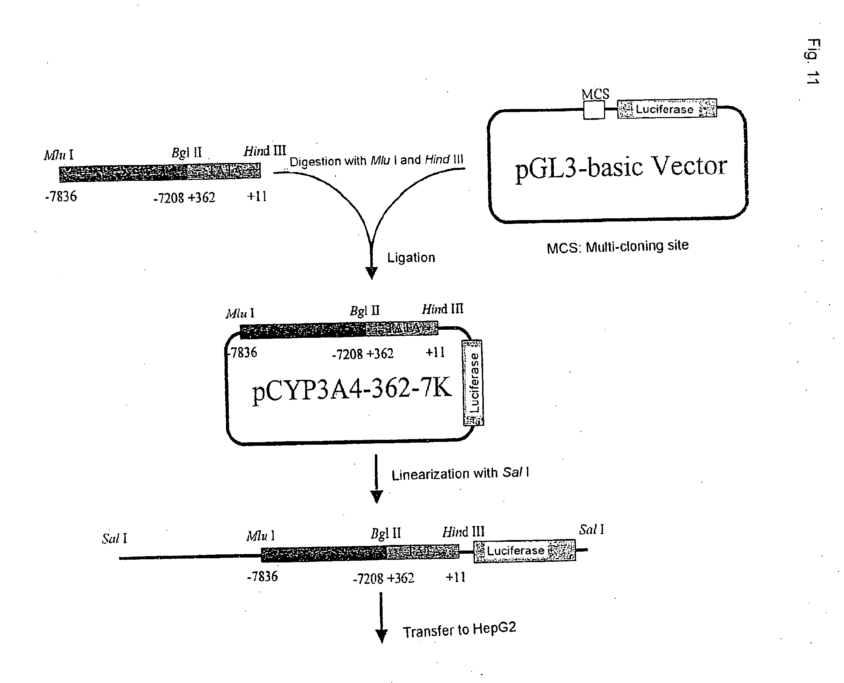

PCR was performed to amplify and isolate a 5'-flanking region +15 to -362 bp and from -7208 bp to -7836 bp of CYP3A4 of human genomic DNA, and inserted to restriction enzyme sites of a pGL3-Basic vector shown in Fig. 11, to thereby create pCYP3A4-362-7K. The resultant pCYP3A4-362-7K was linearized through restriction enzyme treatment with *BamH* I. A plasmid BFP having a neomycin resistant gene was similarly treated with *Bgl* II. pCYP3A4-362-7K and BFP were ligated at a ratio of 5:1. HepG2 cells were transfected with the resultant construct of pCYP3A4-362-7K and BFP by use of the calcium phosphate method. Upon transfection, geneticin was used as a marker for selecting a stable expression cell line. Prior to the use of geneticin, a preliminary test was performed to determine the optimum concentration of geneticin. As a result of the preliminary test, the geneticin concentration to be used was determined to be 900 µg/ml, and selection was carried out. Fourteen days after transfection, generated colonies were transferred one by one to a 24-well plate, and the colonies were incubated in a geneticin-free medium for a further one week. Each colony was placed in an Eppendorf tube containing a suitable amount of medium, the cells were suspended by pipetting, and then transferred to two 24-well plates. When the 24-well plates exhibited confluency of cells, luciferase activity was measured (without use of any inducer). At this point, 8 out of 48 colonies showed activity. The colonies that exhibited activity were subcultured in 10-cm dishes containing a geneticin-free medium. The resultant cells were treated with rifampicin or clotrimazole, serving as an inducer, and luciferase activity was measured. Stocks of the cells were also prepared.

### (2) Cell culture conditions

Cells were seeded in a 24-well plate in an amount of 1.0×10⁵ cells/well. On the following day, the cells were transferred to a medium containing an inducer, followed by incubation for 24 hours. The inducer was diluted with DMSO, and added so as to attain a final concentration of 10 µM. After culturing for two days, the luciferase activity was measured.

### (3) Cell lysis

The medium was removed, followed by washing with PBS. An appropriate amount of PBS was added, and cells were scraped and recovered. The cells were subjected to centrifugation for five minutes at 4°C (15,000 rpm), to thereby remove the supernatant. Subsequently, a reporter lysis buffer (Primega) (100 ml) was added thereto, and the mixture was sufficiently stirred and then stored for at least six hours at -80°C.

### (4) Measurement of luciferase activity

The frozen cell lysate was thawed at room temperature, followed by centrifugation for five minutes at 4°C (15,000 rpm). A reagent, Luciferase Assay System (Promega, 35 µl), was added to the supernatant (20 µl). The mixture was measured through use of a Turner Desingns Luminometer Model TD-20/20 (Promega).

| | |
|---|---|
| Measurement conditions | Delay time: 3 sec |
| | Integrate time: 60 sec |
| | Sensitivity: 60.1% |

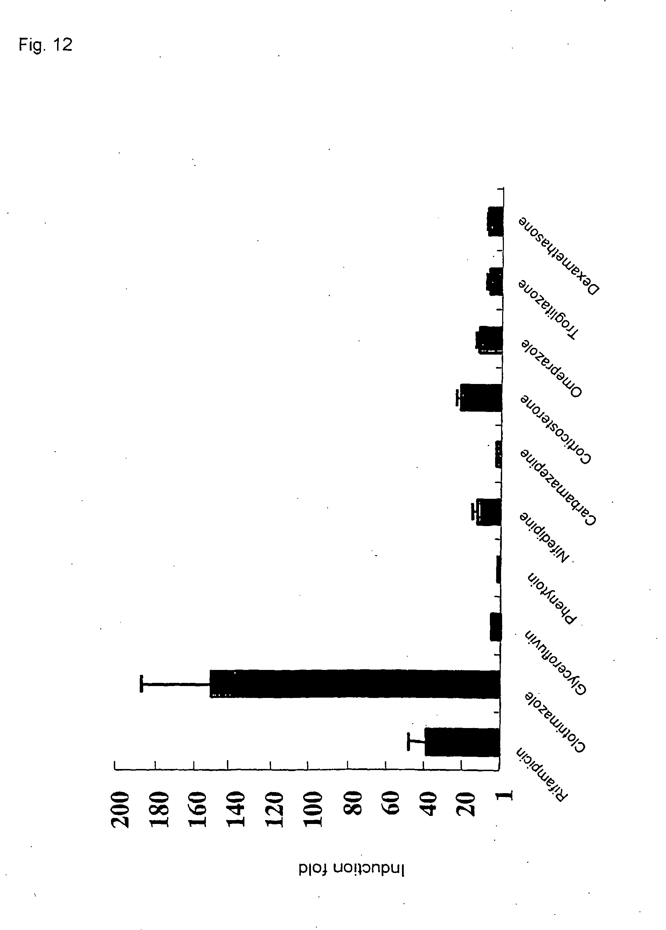

The measurement values were corrected by the protein amount quantified through use of the Bradford method. The results are shown in Fig. 12.

As a result, it has been found that use of transformants which can be subcultured consistently enables a successful assay of human CYP3A inducibility elicited by a variety of drugs.

### INDUSTRIAL APPLICABILITY

The present invention ensures convenient and accurate evaluation of human CYP3A inducibility upon administration of a test drug to a human subject, providing accurate evaluation in terms of the efficacy of the test drug, occurrence of side effects, disappearance of the drug effect, etc.

## Claims

1. A method for measuring human cytochrome P450 3A (CYP3A) inducibility upon administration of a test drug, **characterized in that** either
(a) a population of non-embryonic human cells cultured in a medium containing a test drug is infected with viruses (A) and (B) in such a manner that their functions are exhibited simultaneously;
(i) virus (A) being an adenovirus which is used as a vector and engineered by incorporating a detectable reporter gene and at least 3 human pregnane X receptor (PXR) binding regions falling within an untranslated region of a human CYP3A gene, wherein said regions include at least a 7.7 k (dNR-1) region, a 362 (ER-6) region, and a 7.6 to 7.4 (MIE) region, and
(ii) virus (B) being an adenovirus which is used as a vector and engineered by incorporating a human PXR cDNA;
and subsequently expression level of the reporter gene is determined in the cultured non-embryonic human cells; or
(b) transformed non-embryonic human cells are cultured in a medium containing a test drug, the transformed human cells being created by means of transfer of DNA, wherein the DNA is constructed by inserting, into a plasmid vector, a detectable reporter gene and at least 3 human PXR binding regions falling within an untranslated region of a human CYP3A gene, wherein said regions include at least a 7.7 k (dNR-1) region, a 362 (ER-6) region, and a 7.6 to 7.4 (MIE) region; and then the expression level of the reporter gene is measured.

2. A reagent for measuring human CYP3A inducibility, comprising viruses (A) and (B); wherein virus (A) is an adenovirus which is engineered by incorporating a detectable reporter gene and at least 3 human PXR binding regions falling within an untranslated region of a human CYP3A gene, wherein said regions include at least a 7.7 k (dNR-1) region, a 362 (ER-6) region, and a 7.6 to 7.4 (MIE) region, and virus (B) is an adenovirus, which is engineered by incorporating a human PXR cDNA.

## Patentansprüche

1. Verfahren zu Messung der Induzierbarkeit von menschlichem Cytochrom P450 3A (CYP3A) nach Verabreichung eines Testwirkstoffs, **dadurch gekennzeichnet, dass** entweder
(a) eine Population nichtembryonischer menschlicher Zellen, die in einem einen Testwirkstoff enthaltenden Medium kultiviert wird, mit Viren (A) und (B) in solcher Weise infiziert wird, dass deren Funktionen simultan gezeigt werden;
(i) Virus (A) ist ein Adenovirus, das als Vektor verwendet wird und hergestellt wird durch Einverleiben eines detektierbaren Reportergens und mindestens 3 menschlichen Pregnan X Rezeptor (PXR)-Bindungsregionen, die in eine untranslatierte Region eines menschlichen CYP3A-Gens fallen, wobei diese Regionen mindestens eine 7.7 k (dNR-1) Region, eine 362 (ER-6) Region und eine 7.6 bis 7.4 (MIE) Region enthalten, und
(ii) Virus (B) ist ein Adenovirus, das als Vektor verwendet wird und hergestellt wird durch Einverleiben einer menschlichen PXR cDNS;
sowie anschließender Bestimmung des Expressionsniveaus des Reportergens in den kultivierten nichtembryonischen menschlichen Zellen; oder
(b) Kultivierung von transformierten nichtembryonischen menschlichen Zellen in einem eine Testverbindung enthaltenden Medium, wobei die transformierten menschlichen Zellen erzeugt wurden durch Transfer von DNS, wobei die DNS hergestellt wurde durch Insertieren in einen Plasmidvektor eines detektierbaren Reportergens und mindestens 3 menschlichen PXR Bindungsregionen, die in eine untranslatierte Region eines menschlichen CYP3A-Gens fallen, wobei diese Regionen mindestens eine 7.7 k (dNR-1) Region, eine 362 (ER-6) Region, und eine 7.6 bis 7.4 (MIE) Region beinhalten und anschließend Messung des Expressionsniveaus des Reportergens.

2. Reagenz zur Messung menschlicher CYP3A-Induzierbarkeit, umfassend Virus (A) und (B), wobei Virus (A) ein Adenovirus ist, das hergestellt wurde durch Einverleiben eines detektierbaren Reportergens und mindestens 3 menschlicher PXR Bindungsregionen, die in eine untranslatierte Region eines menschlichen CYP3A-Gens fallen, wobei diese Regionen mindestens eine 7.7 k (dNR-1) Region, eine 362 (ER-6) Region, und eine 7.6 bis 7.4 (MIE) Region beinhalten, und Virus (B) ein Adenovirus ist, das hergestellt wurde durch Einverleiben einer menschlichen PXR cDNS.

## Revendications

1. Procédé pour mesurer l'inductibilité du cytochrome humain P450 3A (CYP3A) lors de l'administration d'un médicament de test, **caractérisé en ce que** soit :
(a) une population de cellules humaines non embryonnaires cultivées dans un milieu contenant un médicament de test est infectée par des virus (A) et (B) de telle manière que leurs fonctions se manifestent simultanément ;
(i) le virus (A) étant un adénovirus utilisé en tant que vecteur et conçu par incorporation d'un gène rapporteur détectable et d'au moins 3 régions de liaison au récepteur X de prégnane (PXR) humain situées à l'intérieur d'une région non traduite d'un gène de CYP3A humain, lesdites régions contenant au moins une région de 7, 7 k (dNR-1), une région de 362 (ER-6) et une région de 7,6 à 7,4 (MIE), et
(ii) le virus (B) étant un adénovirus utilisé en tant que vecteur et conçu par incorporation d'un ADNc de PXR humain ;
et ensuite le niveau d'expression du gène rapporteur est déterminé dans les cellules humaines non embryonnaires cultivées ; soit
(b) des cellules humaines non embryonnaires transformées sont cultivées dans un milieu contenant un médicament de test, les cellules humaines transformées étant créées au moyen d'un transfert d'ADN, l'ADN étant assemblé par insertion, dans un vecteur plasmidique, d'un gène rapporteur détectable et d'au moins 3 régions de liaison au PXR humain situées à l'intérieur d'une région non traduite d'un gène de CYP3A humain, lesdites régions contenant au moins une région de 7,7 k (dNR-1), une région de 362 (ER-6) et une région de 7,6 à 7, 4 (MIE) ; et ensuite le niveau d'expression du gène rapporteur est mesuré.

2. Réactif pour mesurer l'inductibilité du CYP3A humain, comprenant des virus (A) et (B) ; dans lequel le virus (A) est un adénovirus conçu par incorporation d'un gène rapporteur détectable et d'au moins 3 régions de liaison au PXR humain situées à l'intérieur d'une région non traduite d'un gène de CYP3A humain, lesdites régions contenant au moins une région de 7,7 k (dNR-1), une région de 362 (ER-6) et une région de 7,6 à 7,4 (MIE), et le virus (B) est un adénovirus conçu par incorporation d'un ADNc de PXR humain.
